# EUROPEAN PATENT APPLICATION

(11) **EP 3 034 020 A1**
(43) Date of publication of application: **22.06.2016**
(21) Application number: 15167467.8
(22) Date of filing: 13.05.2015
(51) Int. Cl.: A61B 17/34, A61B 5/00

(54) **IMPLANTATION KIT FOR IMPLANTATION OF AN IMPLANTABLE MEDICAL DEVICE**

(30) Priority: 15.12.2014 US 201462091650 P
(71) Applicant: BIOTRONIK SE & Co. KG, 12359 Berlin (DE)
(72) Inventor: Suwito, Wantjinarjo, West Linn, OR 97068 (US); Kraetschmer, Hannes, West Linn, OR 97068 (US); von Arx, Jeffrey A., Lake Oswego, OR 97035 (US); Müssig, Dirk, West Linn, OR 97068 (US); Stotts, Larry, Tigard, OR 97224 (US)
(74) Representative: Galander, Marcus

(57) **Abstract**

An implantation kit that may be used for creating a subcutaneous implant pocket and implanting an implantable medical device therein, is disclosed. The implantation kit includes an implantable medical device (100), a tunneling device (200), and an insertion tool (300).

## Description

The present invention generally relates to a kit comprising special implant tools that may be used with the method.

Implantable medical devices are widely used in the medical field to replace, enhance, or monitor biological structures and biological functions. Some implantable medical devices act autonomously, some work in conjunction with other medical devices, and some function as a hub for several other medical devices. A particular type of implantable medical device is a monitoring device, which may be used to measure and/or sample body signals. Such devices are commonly referred to as implantable monitoring devices. Implantable monitoring devices are typically implanted subcutaneously in a region of the body where they can measure and/or sample relevant body signals. For example, monitoring devices used to measure ECG signals may be implanted in the upper body region near the heart.

Implantable monitoring devices may be configured to measure and/or sample body signals directly, while others are configured to measure and/or sample body signals received/transmitted from other apparatuses, implants, and sensors. Some of these apparatuses may be external to the body, such as a patch for example. The body signals measured from implantable monitoring devices may be measured continuously in real time, sampled from the signal, or acquisitioned from stored data representative of the signal.

Implantable medical devices may comprise a rigid housing containing sensors and other circuitry, or at least facilitating the incorporation thereof. Such devices range in size and shape. Larger sized implantable medical devices, such as, for example, pacemakers and defibrillators, may be inserted by hand without use of a special implant tool; but doing so may be cumbersome and inconvenient. The difficultly of implanting smaller implantable medical devices without a special implant tool increases due to their size and shape and the nature of the implant procedure. Moreover, implantation of implantable medical devices that have flexible housings may become problematic if performed without use of a special implant tool.

As disclosed herein, the inventive method for implanting implantable medical devices providing an effective means to accommodate implanting implantable medical devices, ranging from large to small and from rigid to flexible, overcomes and/or solves many of the problems experienced by practitioners performing such implants. Moreover, the method may be used with a disclosed special implant tool kit to provide additional beneficial improvements.

Prior art methods for implanting implantable medical devices generally consist of deploying the implantable medical device by pushing it with a syringe/plunger-style tool to imbed it within the body of the human or animal. With the syringe/plunger-style tool, an implantable medical device is loaded into a cylinder-like holder and pushed forward by a piston actuated by a plunger until it is imbedded subcutaneously into the body. Such prior art implant methods and tools suffer from many disadvantages that the presently disclosed implantation method and kit do not have, and which the implantation method and kit overcome, and/or solve.

In particular, prior art implant methods and special implant tools fail to accommodate desired configurations of the implantable medical device, which may include extraordinary shapes and implantable medical devices that are flexible or semi-flexible. A flexible or semi-flexible implantable medical device enables better manipulation and positioning of the device for better sensing capabilities. Yet, prior art implant methods hardly accommodate such implantable medical device features, and prior art implant tools fail to provide an effective means to facilitate their implantation. A particular disadvantage exhibited by the prior art is that while implanting flexible or semi-flexible implantable medical devices, the prior art methods and tools fail to maintain a low profile shape of the implantable medical devices when being inserted through the incision and into the subcutaneous implant pocket of the body. For example, prior art methods and tools for implantation do not prevent the implantable medical device from bending, buckling, or becoming misaligned or askew with the desired orientation when being implanted.

Prior art methods and tools for implantation also fail to provide a means to create an appropriate sized subcutaneous implant pocket for the implantable medical device by ensuring that the subcutaneous implant pocket is large enough to accommodate the implantable medical device, yet not excessively large.

Prior art methods and tools for implantation also fail to ensure that the process of implantation does not inadvertently enlarge or cause abrasion to the incision site.

Prior art methods and tools for implantation also fail to provide a means to ensure that creation of the subcutaneous implant pocket and/or subsequent implantation of the implantable medical device does not cause abrasion to the muscle or other tissue.

Prior art methods and tools for implantation also fail provide a means to ensure that the implantable medical device is not inadvertently implanted into the muscle or other tissue at a non-desired location.

Thus, there is a technical need for a method , a kit, and a tool for implantation that accommodate implantation of an implantable medical device that may be rigid, semi-flexible, or flexible, while ensuring a low profile and proper alignment of the implantable medical device during insertion through an incision site and deployment into a subcutaneous implant pocket. There is also a technical need for a solution to enable implantation of the implantable medical device so as to ensure minimal incision and minimal pocket size formation, while preventing inadvertent abrasion and other injury to the incision site and underlying muscle.

The present invention is directed toward overcoming one or more of the above-identified problems.

The presently disclosed invention is directed towards a method for implanting an implantable medical device into the body of a human or animal, which may be performed using the inventive kit that includes an implantable medical device, a tunneling device, and an insertion tool.

The method for implantation of an implantable medical device with an implantation kit comprises the steps of: creating an opening through a skin's surface; creating a subcutaneous implant pocket with a tunneling device; implanting the implantable medical device into the subcutaneous implant pocket with an insertion tool; and, closing the opening of the skin with the implantable medical device within the subcutaneous implant pocket. Creating the opening through a skin's surface comprises creating an incision that is sufficiently long to insert a tunneling distal portion of the tunneling device along its longitudinal axis. Creating the subcutaneous implant pocket with the tunneling device further comprises: gripping the tunneling device; inserting the tunneling device through the incision; and, removing the tunneling device by extracting it through the incision. Gripping the tunneling device comprises gripping the tunneling device with a first hand of a user. Implanting the implantable medical device into the subcutaneous implant pocket with the insertion tool further comprises: gripping the insertion tool; inserting the insertion tool through the incision; and, removing the insertion tool by extracting it through the incision. Gripping the insertion tool comprises gripping the insertion tool with a first hand of the user. The implantable medical device may be mounted onto the insertion tool (*i.e.,* preloaded) before gripping the insertion tool.

The implantable medical device can include a housing that contains electrical circuitry for replacing, enhancing, or monitoring biological functions. The housing may be rigid, semi-flexible, or flexible. The flexible nature of some embodiments enables increased functionality and better conformity with anatomical parts when inserted into the body.

The tunneling device is configured to create an appropriate sized subcutaneous implant pocket after an incision has been made into the skin of the body. The tunneling device may be used and manipulated by a single hand. The tunneling device is provided with a tunneling stopper to assist a user in manipulating the device and to prevent over-insertion and/or under-insertion so that the appropriate sized subcutaneous implant pocket is formed. Minimizing the size of the subcutaneous implant pocket for implantation significantly reduces scaring and risk of infection.

The insertion tool is configured to support the implantable medical device while it and the implantable medical device are inserted into the subcutaneous implant pocket created by the tunneling device. The insertion tool may be used and manipulated by a single hand. The insertion tool is configured to adequately support an implantable medical device in a position that is conducive for implantation into the subcutaneous implant pocket, regardless of whether the implantable medical device is rigid, semi-flexible, or flexible. The insertion tool is further configured to enable a user to retain the implantable medical device with a user's thumb or other finger while the implantable medical device is mounted on the insertion tool, but also enable manipulation and movement of the implantable medical device with the thumb or other finger.

After an incision is made in the skin of a body, the tunneling device is used to create a subcutaneous implant pocket between the skin and the muscle. A user employs their thumb by pressing on the tunneling stopper to assist with supporting the tunneling device and with advancing a distal portion of the tunneling device into the incision. The tunneling stopper also prevents the user from over-inserting and/or under-inserting the tunneling device by abutting the skin of the patient to indicate that insertion is complete. After the subcutaneous implant pocket is created, the tunneling device is removed from the incision.

The implantable medical device is placed or preloaded on an implant holder portion of the insertion tool. A user grasps a proximal portion of the insertion tool with their hand, and inserts a distal portion thereof, along with the implantable medical device mounted thereon, through the incision and into the subcutaneous implant pocket. Upon removal of the insertion tool, the implantable medical device is left to remain inside the subcutaneous implant pocket.

Both the method of implantation and the tools of the implantation kit enable implantation while preventing inadvertent abrasion or other injury to the incision site and underlying muscle during implantation. Both the method and the tools of the implantation kit enable implantation of specially configured implantable medical devices, including those that are flexible and semi-flexible. The components of the implantation kit and the method of use disclosed herein enable quick and effective implantation of an implantable medical device while maintaining a low profile and proper alignment of the implantable medical device, minimizing the required incision size, minimizing the subcutaneous pocket size formation, preventing inadvertent abrasion or injury, and/or reducing scaring and risk of infection.

While these potential advantages are made possible by technical solutions offered herein, they are not required to be achieved. The presently disclosed implantation kit and method of use can be implemented to achieve technical advantages, whether or not these potential advantages, individually or in combinations, are sought or achieved.

Further features, aspects, objects, advantages, and possible applications of the present invention will become apparent from a study of the exemplary embodiments and examples described below, in combination with the Figures, and the appended claims.

The above and other objects, aspects, features, advantages and possible applications of the present invention will be more apparent from the following more particular description thereof, presented in conjunction with the following drawings, in which:
- FIG. 1: illustrates a first embodiment of an implantation kit including a tunneling device, an insertion tool, and an implantable medical device, in accordance with the present disclosure;
- FIG. 2: illustrates a second embodiment of an implantation kit including a tunneling device, an insertion tool, and an implantable medical device in accordance with the present disclosure; and,
- FIGS. 3A and 3B: are illustrative steps of a method for implanting an implantable medical device, in accordance with the present disclosure.

The following description is of an embodiment presently contemplated for carrying out the present invention. This description is not to be taken in a limiting sense, but is made merely for the purpose of describing the general principles and features of the present invention. The scope of the present invention should be determined with reference to the claims.

The present disclosure describes a method of implantation of an implantable medical device 100, 1000, which may be used in conjunction with the disclosed implantable kit 10, 20.

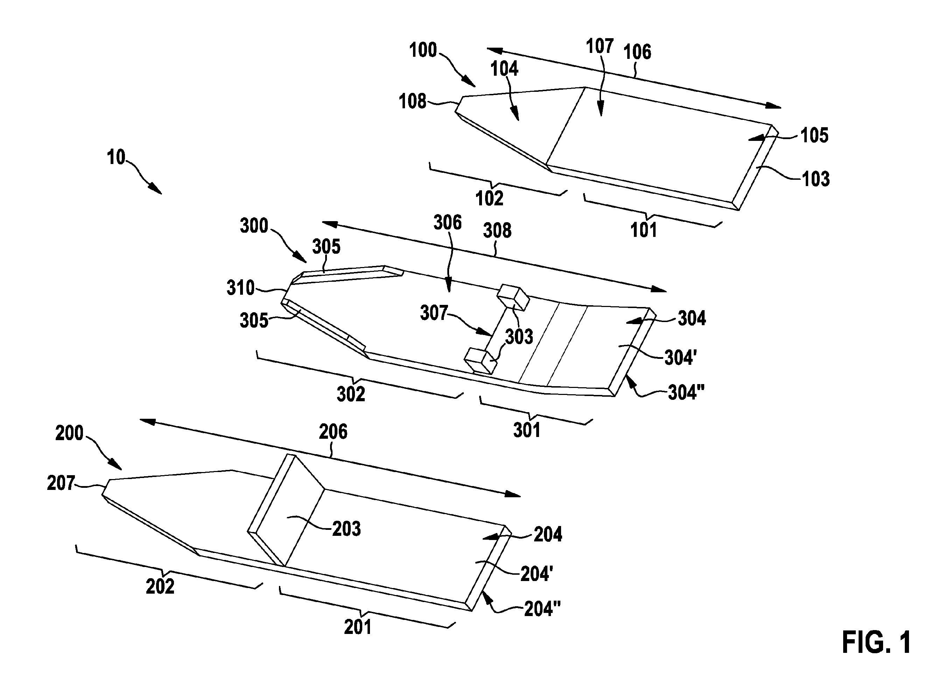

Referring now to FIG. 1, a first embodiment of an implantation kit 10 including a tunneling device 200, an insertion tool 300, and an implantable medical device 100 is disclosed.

The implantation kit 10 comprises an implantable medical device 100, a tunneling device 200, and an insertion tool 300. While the kit 10 is described herein as including an implantable medical device 100, a tunneling device 200, and an insertion tool 300, the kit 10 may include more or less of these components. For example, the kit 10 may comprise the tunneling device 200, the insertion tool 300, and multiple implantable medical devices 100. As another example, the kit 10 may comprise the tunneling device 200 and the insertion tool 300. As another example, the kit 10 may comprise the insertion tool 300 and the implantable medical device 100.

The implantable medical device 100 may be an implantable monitoring device in the form of a subcutaneous monitoring device. While the implantable medical device 100 may be described as one that is used to replace, enhance, or monitor biological structures or biological functions, it is understood that other implantable medical devices may be used. The implantable medical device 100 includes any device configured to be implanted into the body of a human or animal.

The implantable medical device 100 comprises a housing 107 configured to contain sensors and other electrical circuitry, or at least facilitate incorporation thereof. The housing 107 may be a rigid structure, or may be a flexible structure. The housing 107 may include a combination of rigid and flexible structures to enable manipulation of the shape of the implantable medical device 100. The configuration of the housing 107 may enable flexibility so as to provide a resiliency to the device 100. The configuration of the housing 107 may provide for manual and/or automatic manipulation of the shape to enable contorting the implantable medical device 100 into a desired shape. One skilled in the art, with the benefit of the present disclosure, will appreciate that other configurations of rigid, flexible, and semi-flexible portions may be utilized without deviating from the teachings of the implantable medical device 100. Materials used for the housing may include polymers, metal-alloys, shape-memory material, shape-changing material, softening material, or any combination thereof.

The implantable medical device 100 has a device proximal portion 101 and a device distal portion 102. The device proximal portion 101 may house a control unit (not shown) and electrical circuitry (not shown), both of which are in electrical connection with a first electrically active area 105 of the device proximal portion 101. The first electrically active area 105 may be located on a surface of the device proximal portion 101. The first electrically active area 105 can be an electrode disposed on the housing of the device proximal portion 101. The control unit and electrical circuitry are configured for telemetric signal transmission. The device proximal portion 101 includes a device proximal end 103 at its terminus.

The device distal portion 102 may house an electrical connector (not shown) in electrical connection with at least one second electrically active area 104. The second electrically active area 104 may be located on a surface of the device distal portion 102. The second electrically active area 104 can be an electrode disposed on the surface of the device distal portion 102. In one form, the electrical connector comprises a telemetry antenna. In one form, the device distal portion 102 has a tapered configuration, wherein it is most narrow at the distal end 108 of the device distal portion 102.

While the device proximal portion 101 is described as including the control unit and electrical circuitry, and the device distal portion 102 is described as including the electrical connector, one skilled in the art, with the benefit of the present disclosure, will appreciate that the device proximal portion 101 may include the electrical connector and the device distal portion 102 may include the control unit and electrical circuitry.

The control unit, electrical circuitry, electrical connector, and electrically active areas 104, 105 may comprise the necessary components to transmit, receive, store, and acquisition analog and digital signals and perform signal processing. This may include, but is not limited to, circuit boards, memory, transmitters, receivers, transceiver circuitry, converters, amplifiers, batteries, etc.

The implantable medical device 100 is configured to be an elongated structure that extends along a longitudinal axis 106. However, any portion of the implantable medical device 100 may be flat, conical, curvilinear, angled, or exhibit other contours and shapes. The implantable medical device 100 may comprise other components to perform other functions. These may include, but are not limited, to data storage units, sensors, pumps, commutators, etc. Such circuitry and accompanying components may be rigid, flexible, or semi-flexible as well. For example, any of the electrical components may include a flexible hardware platform.

The tunneling device 200 includes an elongated rigid member. While the tunneling device 200 is rigid, it may exhibit some flexibility and resiliency. The tunneling device 200 has a tunneling proximal portion 201 extending along a longitudinal axis 206 to a tunneling distal portion 202. The tunneling device 200 can be fabricated from lightweight material. This may include, but is not limited to plastic, metal, ceramic, etc. The tunneling device 200 may be a unitary piece or may be configured as a combination of multiple interconnecting pieces. The interconnecting pieces may be configured to interconnect via a snap fit, interference fit, etc.

The tunneling proximal portion 201 has a gripping portion 204. The gripping portion 204 includes a gripping portion top side 204' and a gripping portion bottom side 204". The gripping portion 204 may be textured or provided with a non-slip coating to provide added dexterity. The tunneling proximal portion 201 may be flat, conical, curvilinear, straight, or exhibit other contours and shapes.

The tunneling distal portion 202 is configured to exhibit a taper, wherein it is most narrow at the distal end 207 of the tunneling distal portion 202. The taper end enables creation of a tapered subcutaneous implant pocket during implantation of the implantable medical device 100 while using the kit 10 in the manner described by the disclosed method. The tapered subcutaneous implant pocket may be exploited by a practitioner to assist with alignment of the implantable medical device 100 when it is inserted subsequent the creation of the tapered subcutaneous implant pocket. The taper of the tunneling distal portion 202 may be a continuous gradient from the distal end to the tunneling stopper 203, or may be formed by a plurality of different pitched gradients from the distal end to the tunneling stopper 203, or may be formed by a plurality of stepped segments exhibiting successive increases in width from the distal end to the tunneling stopper 203. The taper may extend the entire length of the tunneling distal portion 202 or may appear only on a portion thereof. One skilled in the art, with the benefit of the present disclosure, will appreciate that other profiles in addition to a tapered end may be utilized to assist a practitioner with alignment of the implantable medical device 100 when it is inserted subsequent the creation of the subcutaneous implant pocket. In addition, the tunneling distal portion 202, or any portion thereof, may be straight, angled, curvilinear, flat, conical, etc.

An tunneling stopper 203 is provided on a top surface of the tunneling distal portion 202. The tunneling stopper 203 is a protrusion extending from the top surface, and which can be located so as to demarcate the separation between the tunneling proximal portion 201 and the tunneling distal portion 202. The tunneling stopper 203 may extend perpendicularly from the top surface, or may extend at an angle. The tunneling stopper 203 is configured to provide dexterity and support when a user places a finger against the tunneling stopper 203 while the tunneling device 200 is inserted into the incision site in a manner described by the disclosed method. As will be described by the disclosed method, the tunneling stopper 203 may serve as a stopper to prevent over-insertion and/or under-insertion by abutting the skin at the incision when the tunneling device 200 is inserted into the incision.

The shape and profile of the tunneling distal portion 202 may be made to complement and accommodate a particular implantable medical device 100 included with the kit. Additionally, the profile of the tunneling distal portion 202 may be made to accommodate the desired alignment of the implantable medical device 100 accompanying the kit 10 upon implantation into the subcutaneous implant pocket. The shape and profile of the tunneling distal portion 202, along with the tunneling stopper 203, ensures that the tunneling device 200 may be used to create an appropriate sized subcutaneous implant pocket. The configuration of the tunneling device 200, along with the method of use described below, ensures that an appropriate sized subcutaneous implant pocket may be created without inadvertent abrasion or other injury to the incision site or underlying muscle. Ensuring that an appropriate sized subcutaneous pocket is formed and preventing inadvertent abrasion minimizes scaring and reduces risk of infection.

The insertion tool 300 includes an elongated rigid member. While the insertion tool 300 is rigid, it may exhibit some flexibility and resiliency. The insertion tool 300 has an insertion proximal portion 301 extending along a longitudinal axis 308 to an insertion distal portion 302. The insertion tool 300 can be configured to exhibit a curvilinear structure to facilitate easy ingress and egress from an incision of a body, as well as provide leverage and improve dexterity during use. The curvilinear shape also enables insertion of the insertion tool 300 and deployment of the implantable medical device 100 without abrasion to the incision site or underlying muscle. One skilled in the art, with the benefit of the present disclosure, will appreciate that other shapes and configurations may be utilized to accommodate deployment of implantable medical devices 100 that may be specially configured or exhibit extraordinary shape and to accommodate compromising anatomical locations of the body. For instance, the insertion tool 300, or any portion thereof, may be straight, angled, flat conical, etc.

The insertion tool 300 can be fabricated from lightweight material. This may include, but is not limited to, plastic, metal, ceramic, etc. The insertion tool 300 may be a unitary piece or may be configured as a combination of multiple interconnecting pieces. The interconnecting pieces may be configured to interconnect via a snap fit, interference fit, etc.

The insertion distal portion 302 is configured to exhibit a taper, wherein it is most narrow at the distal end 310 of the insertion distal portion 302. The tapered end enables precise insertion of the implantable medical device 100 without inadvertently widening the incision or subcutaneous implant pocket when inserted therein. The taper of the insertion distal portion 302 may be a continuous gradient from the distal end to the implant stopper 303, or may be formed by a plurality of different pitched gradients from the distal end to the implant stopper 303, or may be formed by a plurality of stepped segments exhibiting successive increases in width from the distal end to an implant stopper 303. The taper may extend the entire length of the insertion distal portion 302 or may appear only on a portion thereof. One skilled in the art, with the benefit of the present disclosure, will appreciate that other profiles in addition to a tapered end may be utilized to assist a practitioner with alignment of the implantable medical device 100 when it is inserted with, and deployed by, the insertion tool 300. For instance, the insertion distal portion 302, or any portion thereof, may be straight, angled, curvilinear, flat, conical, etc.

The insertion proximal portion 301 includes an insertion gripping portion 304 having a top side 304' and a bottom side 304". The insertion proximal portion 301 is provided with an implant stopper 303. The implant stopper 303 includes two protrusions extending from the top surface of the insertion tool 300 at opposite edges thereof. At least a portion of the surface of the insertion proximal portion 301 may be provided with a textured surface or a non-slip coating to assist with dexterity while using the insertion tool 300.

An implant recess 307 may be formed into the top surface of insertion tool 300, which, in conjunction with the implant stopper 303, forms the implant holder 306. During use, the implantable medical device 100 is placed within the implant holder 306 to be temporarily held in place. The implant stopper 303 is configured to enable motion of a user's finger along the longitudinal axis 308 so as to allow traverse motion between the protrusions. As will be described by the disclosed method, this enables a user to hold and use the insertion tool 300 in one hand while allowing a user's finger to hold the implantable medical device 100 in place or manipulate the position of the implantable medical device 100 by making contact with the device proximal end 103. The implant stopper 303 is also configured to serve as a stopper to prevent over-insertion and/or under-insertion by abutting the skin at the incision when the insertion distal portion 302 is inserted through the incision.

The shape and profile of the insertion distal portion 302 may be made to complement and accommodate a particular implantable medical device 100 included in the kit 10. Additionally, the profile of the insertion distal portion 302 may be made to accommodate the desired alignment of the implantable medical device 100 accompanying the kit 10 upon implantation into the subcutaneous implant pocket. The shape and profile of the insertion distal portion 302, along with the implant stopper 303, ensures that the insertion tool 300 may be used to implant the implantable medical device 100 quickly and effectively while using a single hand to operate the insertion tool 300. The configuration of the insertion tool 300, along with the method of use described below, ensures that deployment of the implantable medical device 100 occurs without inadvertent abrasion or other injury to the incision site or underlying muscle, and occurs so as to implant the implantable medical device 100 in the desired orientation within the subcutaneous implant pocket, especially if the implantable medical device 100 is flexible in any manner.

The configuration of the insertion tool 300 enables mounting the implantable medical device 100 thereon such that the longitudinal axes 106, 308 are parallel. Therefore, when the insertion tool 300 is used to implant the implantable medical device 100, a practitioner may be assured that the alignment of the implantable medical device 100 will match that of the insertion tool 300. At least this feature alone greatly assists the practitioner to ensure proper alignment of the implantable medical device 100 within the subcutaneous implant pocket.

A top portion of the insertion distal portion 302 is provided with at least one guide mechanism 305. The guide mechanism 305 may be used to assist a practitioner to advance the implantable medical device 100 in a particular direction while the implantable medical device 100 is being deployed into the subcutaneous implant pocket. The guide mechanism 305 may also be used to assist with preventing bending, buckling, and/or misalignment of the implantable medical device 100 when motioning it in a forward or rearward direction during deployment. The guide mechanism 305 may also be used for the same purposes when mounting the implantable medical device 100 onto the insertion tool 300 prior to deploying it into the subcutaneous implant pocket. In one form, the guide mechanism 305 includes a set of bars, wherein each bar is disposed on opposing sides of the insertion distal portion 302. The guide mechanism 305 is located near the distal end of the insertion distal portion 302.

The shape and profile of the guide mechanism 305 may be made to complement and accommodate a particular implantable medical device 100 included with the kit 10. The profile of the guide mechanism 305 may be made to accommodate the desired alignment of the implantable medical device 100 accompanying the kit 10 upon implantation into the subcutaneous implant pocket. The shape and profile of the guide mechanism 305 assists with implanting the implantable medical device 100 using one hand in a quick and effective manner. The configuration of the guide mechanism 305, along with the method of use described below, assists with deployment of the implantable medical device 100 without inadvertent abrasion or other injury to the incision site or underlying muscle, and with implanting the implantable medical device 100 in the desired orientation within the subcutaneous implant pocket, especially if the implantable medical device 100 is flexible in any manner. On the other side the configuration of the guide mechanism 305, along with the method of use described below, assists with disposing of the implantable medical device 100 within the subcutaneous implant pocket, especially during withdrawing the insertion tool 300 from the subcutaneous implant pocket without unintentionally pulling out the implantable medical device 100. In some embodiments the guide mechanism 305 is tapered at its proximal end or has tapered edges at its proximal end, so that when the insertion tool 300 is withdrawn from the subcutaneous implant pocket, the guide mechanism slides behind the implantable medical device 100. In some embodiments, the guide mechanism 305 may be configured to temporarily retain the implantable medical device 100 via an interference or other fit.

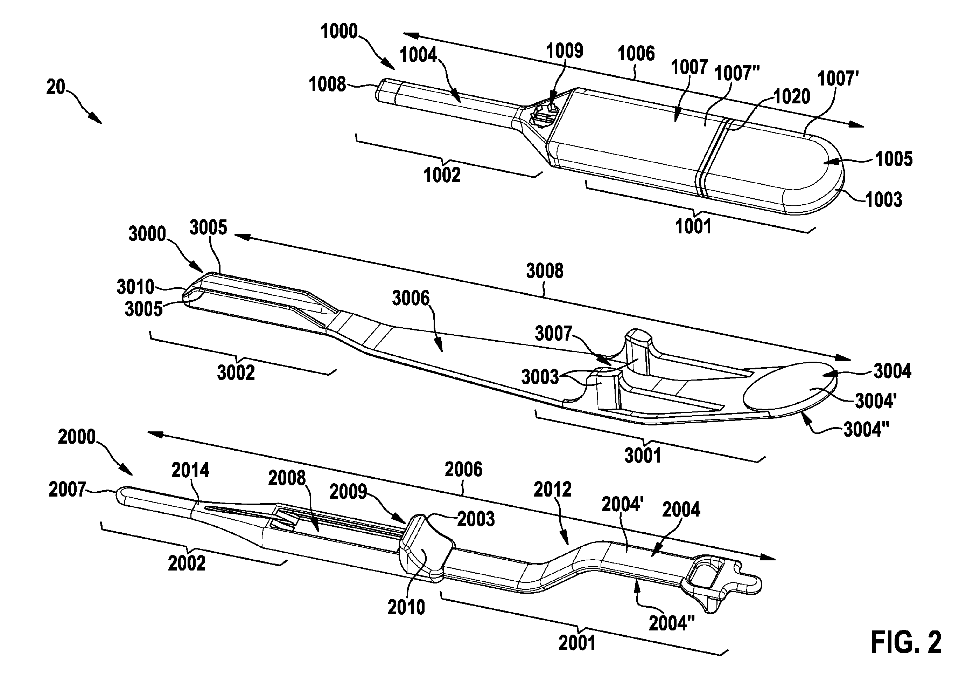

Referring now to FIG. 2, a second embodiment of an implantation kit 20 including a tunneling device 2000, an insertion tool 3000, and an implantable medical device 1000 is disclosed.

In the second embodiment, the implantable medical device 1000 includes a housing 1007 that may be a unitary housing or may include two rigid structures 1007', 1007" connected via a connection 1020. The connection 1020 may be rigid or flexible. The implantable medical device 1000 includes a device proximal portion 1001 with a first electrically active area 1005, and a device distal portion 1002 with a second electrically active area 1004, which may be flexible or rigid, with both portions extending along a longitudinal axis 1006. The device 1000 further includes a device proximal end 1003 and a device distal end 1008. The implantable medical device 1000 exhibits a more rounded shape at its edges than the embodiment shown in FIG. 1, which may reduce any tendency to catch on portions of the subcutaneous implant pocket. The second electrically active area 1004 is configured as an elongated structure extending from a tapered neck 1009 of the device distal portion 1002. The second electrical area 1004 has at or close to the device distal end 1008 a second electrically active area or an electrically active electrode.

The tunneling device 2000 includes a tunneling proximal portion 2001 and a tunneling distal portion 2002 extending along a longitudinal axis 2006. The tunneling device may be rigid or exhibit some flexibility and resiliency, and may be made of the same or similar materials and construction as tunneling device 200.

The tunneling proximal portion 2001 has a gripping portion 2004, which includes a gripping portion top side 2004' and a gripping portion bottom side 2004" (which may or may not have a textured surface for gripping). An tunneling stopper 2003 is provided between the proximal 2001 and distal 2002 portions, and may include an inclined or arcuate back portion 2010 and a flat back portion 2009. The back portion 2010 is designed to engage the thumb of a user and may have a textured surface. The tunneling stopper 2003 may also serve as a stopper to prevent over-insertion and/or under-insertion by abutting the skin at the incision when the tunneling device 2000 is inserted into the incision.

A recess 2008 may also be formed in the tunneling device 2000 adjacent the back portion 2009. The recess 2008 may provide structural rigidity to the tunneling device 2000. As shown in FIG. 2, tunneling proximal portion 2001 may be configured to include an angled portion 2012 to provide dexterity and leverage for a user while using the tunneling device 2000. Additionally, the tunneling distal portion 2002 may include a tapered portion 2014 and a distal end 2007.

The insertion tool 3000 may include a rigid member, or may exhibit flexibility and resiliency. The insertion tool 3000 includes an insertion proximal portion 3001 extending along a longitudinal axis 3008 to an insertion distal portion 3002. Similar to insertion tool 300, the insertion tool 3000 may also have a curvilinear shape and may be fabricated from a lightweight material.

The insertion distal portion 3002 is configured to exhibit a taper (similar to insertion tool 300), wherein it is most narrow at the distal end 3010 of the insertion distal portion 3002. The tapered end enables precise insertion of the implantable medical device 1000 without inadvertently widening the incision or subcutaneous implant pocket when inserted therein. Of course, other non-tapered profiles may also be implemented.

The insertion proximal portion 3001 includes an insertion gripping portion 3004 having a top side 3004' and a bottom side 3004". The insertion proximal portion 3001 is provided with an implant stopper 3003. The implant stopper 3003 includes two protrusions extending from the top surface of the insertion tool 3000 at opposite edges thereof. At least a portion of the surface of the insertion proximal portion 3001 may be provided with a textured surface or a non-slip coating to assist with dexterity while using the insertion tool 3000. As shown in FIG. 2, insertion tool 3000 at or close to the implant stopper 3003 may be configured to include an angle to raise the insertion proximal portion with the insertion gripping portion 3004 and therefore to provide dexterity and leverage for a user while using the insertion tool 3000.

An implant recess 3007 may be formed into the top surface of insertion tool 3000, which, in conjunction with the implant stopper 3003, forms the implant holder 3006. During use, the implantable medical device 1000 is placed within the implant holder 3006 to be temporarily held in place. The implant stopper 3003 is configured to enable motion of a user's finger along the longitudinal axis 3008 so as to allow traverse motion between the protrusions. This enables a user to hold and use the insertion tool 3000 in one hand while allowing a user's finger to hold the implantable medical device 1000 in place or manipulate the position of the implantable medical device 1000 by making contact with the device proximal end 1003. The implant stopper 3003 is also configured to serve as a stopper to prevent over-insertion and/or under-insertion by abutting the skin at the incision when the insertion distal portion 3002 is inserted through the incision.

The shape and profile of the insertion distal portion 3002 may be made to complement and accommodate a particular implantable medical device 100 included in the kit 10, as well as its desired alignment within the subcutaneous implant pocket. The shape and profile of the insertion distal portion 3002, along with the implant stopper 3003, ensures that the insertion tool 3000 may be used to implant the implantable medical device 1000 quickly and effectively while using a single hand to operate the insertion tool 3000, while reducing injury at the incision/implantation site.

The configuration of the insertion tool 3000 enables mounting the implantable medical device 1000 thereon such that the longitudinal axes 1006, 3008 are parallel. Therefore, when the insertion tool 3000 is used to implant the implantable medical device 1000, a practitioner may be assured that the alignment of the implantable medical device 1000 will match that of the insertion tool 3000. At least this feature alone greatly assists the practitioner to ensure proper alignment of the implantable medical device 1000 within the subcutaneous implant pocket.

A top portion of the insertion distal portion 3002 is provided with at least one guide mechanism 3005 at the distal end 3010, which includes a set of bars and is designed to function in a manner previously described with respect to the guide mechanism 305. Generally, the kit 10, 20 is configured so that it may be employed by a physician or other medical personnel to implant the implantable medical device 100, 1000 into the body of a patient. After an incision is made in the skin of the body of the patient (e.g., with a scalpel), the tunneling device 200, 2000 is used to create a subcutaneous implant pocket which is in the shape of the implantable medical device 100, 1000, and only slightly larger. Specifically, the tunneling proximal portion 201, 2001 is grasped by a user (e.g., using their thumb and index finger) to insert the tunneling distal portion 202, 2002 into the incision. The user employs their finger, *e.g.,* their thumb, by pressing on the tunneling stopper 203, 2003 to assist with stabilizing the tunneling device 200, 2000 and with advancing the tunneling distal portion 202, 2002 into the incision. The tunneling stopper 203, 2003 also prevents the user from over-inserting and/or under-inserting the tunneling device 200, 2000 by abutting the skin of the patient to indicate that insertion is complete. After the subcutaneous implant pocket is created, the tunneling device 200, 2000 is removed from the incision.

The implantable medical device 100, 1000 is placed within the implant holder 306, 3006 portion of the insertion tool 300, 3000 (*i.e.,* is preloaded), which may include placing the implantable medical device 100, 1000 within the guide mechanism 305, 3005. The user grasps the insertion proximal portion 301, 3001 with their hand, and inserts the insertion distal portion 302, 3002, along with the implantable medical device 100, 1000, through the incision and into the subcutaneous implant pocket. The implant stopper 303, 3003 prevents the user from over-inserting and/or under-inserting the insertion tool 300, 3000 by abutting the skin of the patient to indicate that insertion is complete. Upon removal of the insert tool 300, 3000, the implantable medical device 100, 1000 is left to remain inside the subcutaneous implant pocket. The incision is then closed.

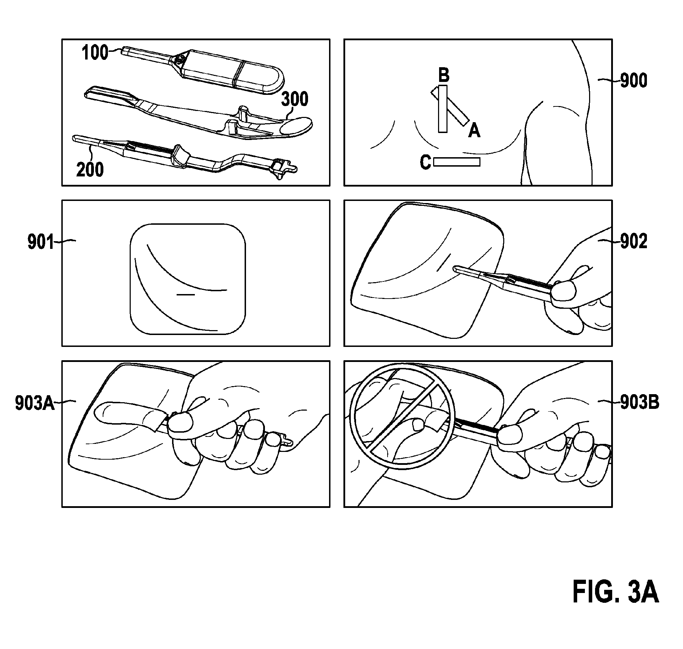

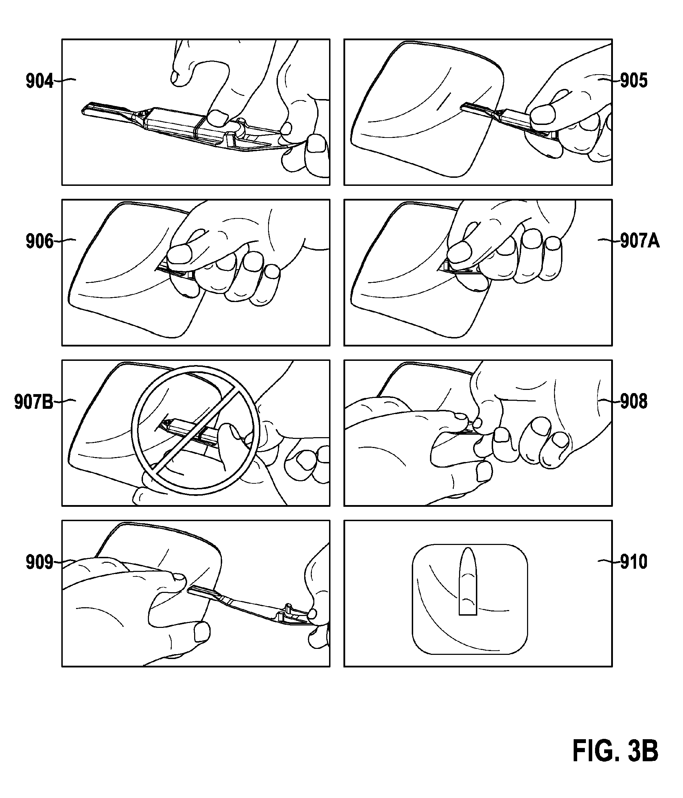

Referring now to FIGS. 3A and 3B, illustrative steps of a method for implanting an implantable medical device, in accordance with the present disclosure, are disclosed.

A method for implanting the implantable medical device 100, 1000 using the kit 10, 20 may include the following steps:
I. Opening the skin, as shown at 901;
II. Creating a subcutaneous implant pocket using the tunneling device 200, 2000, as shown at 902 ,903A and 903B;
III. Inserting the implantable medical device 100, 1000 into the subcutaneous implant pocket using the insertion tool 300, 3000, as shown at 904-909; and,
IV. Closing the skin, as shown at 910. For example, the skin may be closed with a suture or other suitable device or procedure to close the skin.

Prior to step I, the method may include the optional step of determining an implantation site on the skin (e.g., at 900). Opening the skin (step I) may include the step of creating an incision with a scalpel. The incision may be a straight cut in the skin and/or the underlying tissue. The incision should be sufficiently long to enable insertion of the tunneling device 200, 2000 along its longitudinal axis 206, 2006. In one form, the incision may have a length within the range from 5 mm to 20 mm, or a length within the range from 8 mm to 18 mm, or a length within the range from 8 mm to 15 mm. The length may depend on the size of the various devices included within the kit 10, 20.

In one form, step II may include the following steps:
a. Gripping the tunneling device 200, 2000, as shown at 902;
b. Inserting the tunneling device 200, 2000 through the incision and into the body, as shown at 903A; and,
c. Removing the tunneling device 200, 2000 by extracting it through the incision, as shown at 903B.

Gripping the tunneling device 200, 2000 (step II(a)) may include gripping the tunneling device 200, 2000 with a first hand of the user and, specifically, between the thumb and index finger. The tunneling device 200, 2000 is gripped at that gripping portion 204, 2004, which is at the tunneling proximal portion 201, 2001 of the tunneling device 200, 2000. To grip the tunneling device, the user may place the index finger of the first hand on the gripping portion bottom side 204", 2004", and place the thumb of the first hand on the gripping portion top side 204', 2004'. The tunneling proximal portion 201, 2001 may be supported with the remaining fingers of the first hand. Gripping the tunneling device 200, 2000 may also include positioning the index finger of the first hand on the gripping portion bottom side 204", 2004" so as to be opposite of the thumb of the first hand that is placed on gripping portion top side 204', 2001'. The tip of the thumb of the first hand may be placed at the proximal side of the tunneling stopper 203, 2003 to further assist with supporting and manipulating the tunneling device 200, 2000 in a longitudinal direction along its longitudinal axis 206, 2006.

Inserting the tunneling device 200, 2000 (step II(b)) may include inserting the tunneling device 200, 2000 with the tunneling distal portion 202, 2002 first through the incision. The tunneling device 200, 2000 may be inserted subcutaneously as tangentially to the skin surface as possible, for example after passing through the incision in a direction substantially parallel to the skin surface. In one form, the angle of insertion may be from nearly 0-20 degrees, or from 1-20 degrees, or from 1-10 degrees, or from 1-5 degrees with respect to the skin surface. The tunneling device 200, 2000 is pushed forward or advanced in the distal direction until the tunneling stopper 203, 2003 abuts the skin surface to thereby create subcutaneous implant pocket for the implantable medical device 100, 1000. As previously discussed, the manipulation of the tunneling device 200, 2000 may be performed with the first hand of the user; as second hand is not necessary (*see* 903B).

In one form, method step III may include the following steps:
a. Gripping the insertion tool 300, 3000 with the medical device 100, 1000 premounted or preloaded in the implant holder 306, 3006, as shown at 904 and 905;
b. Inserting the insertion tool 300, 3000 through the incision and into the body, as shown at 906, 907A, and 907B; and,
c. Withdrawing the insertion tool 300, 3000 by extracting it through the incision, as shown at 908 and 909.

Gripping the insertion tool 300, 3000 (step III(a)) may include gripping the insertion tool 300, 3000 with one hand (*i.e.,* the first hand). The insertion tool 300, 3000 is gripped between the index finger and thumb, and supported with the remaining fingers of the user. In this manner, the thumb engages the top side 304', 3004', while the index finger engages the bottom side 304", 3004". The insertion tool 300, 3000 may be placed with the bottom side 304", 3004" in the palm of the user's hand and supported by the remaining fingers. The user's thumb may be placed on the device proximal portion 101, 1001 of the premounted implantable medical device 100, 1000 to maintain it in place. The user's index finger is positioned on the bottom side 304", 3004" opposite the thumb on the implantable medical device 100, 1000 to thereby fix the implantable medical device 100, 1000 in the implant holder 306, 3006 of the insertion tool 300, 3000. In this regard, the guide mechanism 305, 3005 helps align the implantable medical device 100, 1000 for insertion.

Inserting the insertion tool 300, 3000 (step III(b)) may include inserting the insertion tool 300, 3000 with the tapered insertion distal portion 302, 3002 first through the incision. The insertion tool 300, 3000 may be inserted subcutaneously into the pre-shaped subcutaneous implant pocket as tangentially to the skin surface as possible, for example substantially parallel as defined by the subcutaneous implant pocket, which is created by the tunneling device 200, 2000, and which is situated substantially parallel to the skin surface. In one form, the angle of insertion may be from nearly 0-20 degrees, or from 1-20 degrees, or from 1-10 degrees, or from 1-5 degrees with respect to the skin surface. The insertion tool 300, 3000 is pushed forward in the distal direction up to the device proximal portion 101, 1001 (i.e., until the implant stopper 303 abuts the skin surface), where the thumb fixes the implantable medical device 100, 1000 in the implant holder 306, 3006 on the insertion tool 300, 3000. The thumb of the user is relocated away from the device proximal portion 101, 1001 of the implantable medical device 100, 1000 to the top gripping side 304', 3004' proximal to the insertion tool's implant stopper 303, 3003 so that the thumb contacts the device proximal end 103, 1003 of the implantable medical device 100, 1000. The insertion tool 300, 3000 is pushed forward through the incision until the implantable medical device 100, 1000 is completely within the subcutaneous implant pocket. The thumb of the user may also be used to help push the implantable medical device 100, 1000 completely within the subcutaneous implant pocket.

Withdrawing the insertion tool 300, 3000 (step III(c)) may include fixing the implantable medical device 100, 1000 in the subcutaneous implant pocket at a location on device the proximal portion 101, 1001 and/or at a location proximal of its device proximal end 103, 1003 to prevent accidental removal of the implantable medical device 100, 1000. In this regard, a finger(s) of an opposite hand of the user may be used to apply pressure to the implantable medical device 100, 1000 to maintain the implantable medical device 100, 1000 in the subcutaneous implant pocket during removal of the insertion tool 300, 3000 *(see* 908 and 909). Otherwise, the implantable medical device 100, 1000 may be withdrawn with the insertion tool 300, 3000 (*see* 907B). The insertion tool 300, 3000 is removed by withdrawing the insertion tool 300, 3000 in the proximal direction along its longitudinal axis 306, 3006 until it is completely removed from the subcutaneous implant pocket, wherein the implantable medical device 100, 1000 is left within the subcutaneous implant pocket.

In some forms, the thumb of the first hand may be moved through the protrusions of the implant stopper 303, 3003 to advance the implantable medical device 100, 1000 in forward or rearward directions and to assist with and positioning the implantable medical device 100, 1000 within the subcutaneous implant pocket and/or disengaging the implantable medical device 100, 1000 from the insertion tool 300, 3000. The thumb of the first hand may also be moved through the protrusions of the implant stopper 303, 3003 to direct the implantable medical device 100, 1000 into and/or out of the guide mechanism 305, 3005.

The inventive method of inserting the implantable medical device 100, 1000 using the tunneling device 200, 2000 and the insertion device 300, 3000 may be performed using only one hand of a user.

The method describing use of the components of the kit 10, 20 with a first hand of a user are exemplary, and are not to be taken as limiting the configurations of the components or the methods of use. A user may use a first hand and a second hand, or a first hand of a first user and a second hand of a second user, or any combination thereof to perform any step of the inventive method utilizing the various kit components. As one example only, a user may use a first hand for gripping the insertion tool 300, 3000, and move a thumb of a second hand through the protrusions of the implant stopper 303, 3003 to advance the implantable medical device 100, 1000 in forward or rearward directions.

## Claims

1. An implantation kit (10, 20) for an implantable medical device (100, 1000), comprising:
a tunneling device (200, 2000), comprising:
a tunneling proximal portion (201, 2001); and
a tunneling distal portion (202, 2002) extending from the tunneling proximal portion; and
an insertion tool (300, 3000), comprising:
an insertion proximal portion (301, 3001) configured to be gripped by the at least one hand of a user;
an insertion distal portion (302, 3002) extending from the insertion proximal portion; and
an implant holder (306, 3006) formed on a top surface of the insertion tool configured to temporarily retain the implantable medical device (100, 1000);
wherein the insertion tool is configured to deploy the implantable medical device into the subcutaneous implant pocket after being inserted therein.

2. The implantation kit (10, 20) recited in claim 1, wherein the insertion distal portion (302, 3002) includes at least one guide mechanism (305, 3005) formed on a top surface thereof configured to temporarily retain the implantable medical device (100, 1000).

3. The implantation kit (10, 20) recited in claim 1, wherein the tunneling device (200, 2000) further comprises a tunneling stopper (203, 2003) formed on a top surface of the tunneling device between the tunneling proximal portion (201, 2001) and the tunneling distal portion (202, 2002).

4. The implantation kit (10, 20) recited in claim 1 or 2, wherein the insertion tool (300, 3000) further comprises an implant stopper (303, 3003) formed on a top surface of the insertion tool.

5. The implantation kit (10, 20) recited in claim 4, wherein the implant stopper (303, 3003) comprises two protrusions extending from the top surface of the insertion tool at opposite edges thereof.

6. The implantation kit recited in at least one of claims 1 to 5, further comprising at least one implantable medical device (100, 1000).
